# EUROPÄISCHE PATENTSCHRIFT

(11) **EP 3 023 784 B1**
(45) Veröffentlichungstag und Bekanntmachung des Hinweises auf die Patenterteilung: **01.03.2017**
(21) Anmeldenummer: 15195702.4
(22) Anmeldetag: 20.11.2015
(51) Int. Cl.: G01N 33/12, G01N 23/083, G01N 23/10, G01V 5/00

(54) **VORRICHTUNG ZUR RÖNTGENUNTERSUCHUNG VON BEWEGTEN PRODUKTEN, INSBESONDERE BEWEGTEN STÜCKGÜTERN**
X-RAY EXAMINATION DEVICE FOR MOVING PRODUCTS, IN PARTICULAR MOVING PIECE GOODS
DISPOSITIF D'EXAMEN RADIOLOGIQUE DE PRODUITS MOBILES, EN PARTICULIER DE MARCHANDISES MOBILES EN VRAC

(30) Priorität: 24.11.2014 DE 102014117196
(43) Veröffentlichungstag der Anmeldung: 25.05.2016
(73) Patentinhaber: Wipotec Wiege- und Positioniersysteme GmbH, 67657 Kaiserslautern (DE)
(72) Erfinder: SCHUFF, Torben, 66606 St. Wendel (DE); THOMAS, Werner, 67691 Hochspeyer (DE)
(74) Vertreter: Eder, Thomas

(56) Entgegenhaltungen:
- JP-A- 2000 241 368
- US-A1- 2005 105 680
- US-A1- 2010 020 927

## Beschreibung

Die Erfindung betrifft eine Vorrichtung zur Röntgenuntersuchung von bewegten Produkten, insbesondere bewegten Stückgütern, mit den Merkmalen des Oberbegriffs des Patentanspruchs 1.

Stückgüter, insbesondere mit einem Medium gefüllte Behälter, werden in Fertigungsstraßen aus Sicherheitsgründen meist nach dem Vorhandensein von Fremdkörpern überprüft. Hierfür geeignete Inspektionssysteme arbeiten dabei häufig mit Röntgenstrahlung, die das Stückgut bzw. den gefüllten Behälter durchdringt, und erzeugen dabei Bilddaten, welche die Erkennung von Fremdkörpern ermöglicht. Auf diese Weise ist selbstverständlich nicht nur die Erkennung von Fremdkörpern möglich, auch Eigenschaften des Stückguts oder des Mediums in dem betreffenden Behälter oder des Behälters selbst können überprüft werden. Beispielsweise können das Vorhandensein und die Position von Knochen in Fleischstücken oder der Fettanteil von Fleischstücken ermittelt werden, da unterschiedliche Materialien oder Stoffe die das Stückgut durchdringende Röntgenstrahlung unterschiedlich absorbieren und damit eine unterschiedliche Dämpfung der Röntgenstrahlung bewirken. Auch der Füllstand eines gefüllten Behälters kann erfasst und beispielsweise auf das Erfüllen einer Mindestfüllhöhe hin überprüft werden.

Derartige Röntgenstrahl-Inspektionsvorrichtungen arbeiten üblicherweise mit einem sich fächerartig von einer Röntgenstrahlungsquelle erweiternden Röntgenstrahl, welcher das zu untersuchende Stückgut durchdringt. Die Röntgenstrahlungsquelle wird hierzu häufig seitlich einer Fördervorrichtung, beispielsweise eines Bandförderers, angeordnet, so dass der fächerartige Röntgenstrahl das Stückgut von der Seite her durchdringt. Auf der anderen Seite der Fördervorrichtung ist eine Detektorvorrichtung vorgesehen, welche die nicht absorbierte Röntgenstrahlung detektiert, nachdem diese das Stückgut durchdrungen hat.

Der fächerartige Röntgenstrahl weist dabei meist eine quer, insbesondere senkrecht zur Förderrichtung verlaufende Strahlebene auf. Die Ausdehnung des Strahls senkrecht zur Strahlebene ist meist gering, jedoch abhängig von der Beschaffenheit der Detektorvorrichtung. Vielfach werden Detektorvorrichtungen in Form einer Detektorzeile verwendet, welche eine Vielzahl von Pixeln aufweist, die äquidistant entlang einer Geraden angeordnet ist. Die Längsachse einer solchen Detektorzeile muss dabei selbstverständlich mit der Strahlebene fluchten. Die Ausdehnung des Röntgenstrahls senkrecht zur Strahlebene muss in diesem Fall nur in etwa der Breite der Pixel der Detektorzeile entsprechen. Ein breiterer Strahl (als Breite sei die Strahlausdehnung senkrecht zur Strahlebene bezeichnet) würde nicht zu einer größeren Empfindlichkeit führen, da die in der Breite des Strahls enthaltene Energie, die nicht mehr von den Pixeln detektiert wird, verloren geht. Bei Verwendung eines Zeilendetektors ist die Auflösung generell durch die Fläche der einzelnen Pixel und den Abstand der Pixel bestimmt. Die Pixelfläche, und damit auch die Pixelbreite, sollte damit in einem Bereich gewählt werden, der gewährleistet, dass die hierdurch erzeugte Auflösung den Ansprüchen an die Detektionsgenauigkeit genügt.

Als spezielle Zeilendetektoren können auch sogenannte Time Delay Integration (TDI-) Detektoren verwendet werden, die zwar mehrere, parallel zur Strahlebene verlaufende Detektorzeilen aufweisen, jedoch als Ausgangssignal das (aufintegrierte) Signal der einzelnen Detektorzeilen liefern. In diesem Fall sollte die Strahlbreite so groß sein, dass alle Zeilen des TDI-Detektors von der Strahlbreite abgedeckt sind.

Bei allen derartigen Zeilenscannern wird das zu untersuchende bewegte Produkt zeilenweise (bzw. scheibchenweise) abgetastet. Das Gesamtbild des Produkts (oder ein Ausschnitt hiervon) kann dann durch das Zusammensetzen der einzelnen Zeilen (die durchstrahlten Scheiben des Produkts entsprechen) als vorzugsweise digitale Information erzeugt werden.

Ganz allgemein kann jedoch auch eine Röntgenkamera als zweidimensional arbeitender Detektor in einer Detektorvorrichtung verwendet werden, wobei die Röntgenkamera hinsichtlich der Detektorfläche so gewählt ist, dass entweder das gesamte Produkt oder ein Ausschnitt hiervon in einem Schritt erfasst wird. Mehrere Bildausschnitte können dabei selbstverständlich ebenfalls wieder zu einem Gesamtbild zusammengesetzt werden. Die Strahlbreite muss in diesem Fall mindestens so groß gewählt werden wie die Breite der empfindlichen Fläche der Kamera, die als zweidimensionaler digitaler Sensor ausgebildet sein kann.

Eine bekannte Vorrichtung der Firma Mettler-Toledo (InspireX R50S) zur Untersuchung von bewegten Produkten verwendet eine Röntgenstrahlungsquelle die seitlich eines Bandförderers angeordnet ist. Die Position der Röntgenstrahlungsquelle fluchtet dabei mit der Oberfläche des Obertrums des Bandes des Bandförderers, auf dem die zu untersuchenden Produkte aufliegen. Hierdurch kann ein Röntgenstrahl erzeugt und verwendet werden, dessen unterer Randstrahl im Wesentlichen parallel zu Bandoberfläche (waagrecht) verläuft. Der untere Bereich des Produkts wird so von einem im Wesentlichen horizontal verlaufenden Teilstrahl (Strahlenbündel mit einem kleinen Öffnungswinkel) durchdrungen. Dies hat dann Vorteile, wenn beispielsweise der Bodenbereich eines Glasbehälters auf Beschädigungen untersucht werden soll. Der seitliche Abstand der Position der Röntgenstrahlungsquelle vom Bandförderer bzw. dem zu untersuchenden Produkt und der Öffnungswinkel des Röntgenstrahls sind so gewählt, dass das gesamte Produkt von dem fächerartigen Strahl durchdrungen wird.

Die Position der Röntgenstrahlungsquelle kann bei dieser Vorrichtung horizontal variiert werden, so dass - bei konstantem Öffnungswinkel des Strahls - der Strahl immer gerade so eingestellt werden kann, dass bei der gegebenen Höhe und Form des Produkts immer die minimal mögliche Strahlhöhe (beispielsweise gemessen an der Position des Produkts, welche den minimalen Abstand zur Röntgenstrahlungsquelle in der Strahlebene aufweist) verwendet wird. Mit anderen Worten, die Röntgenstrahlungsquelle kann so positioniert werden, dass immer die gesamte Strahlungsenergie durch das Produkt hindurchtritt (in der worst case-Ebene des Produkts, welche bei einer Positionierung des Produkts durch den Bandförderer in der Strahlebene den maximalen Strahlwinkel erfordert; in anderen Positionierungen des Produkts kann selbstverständlich ein Teil der Strahlungsenergie am Produkt vorbei gehen). Für die Untersuchung von Produkten mit kleiner Höhe kann die Röntgenstrahlungsquelle also nahe an den Bandförderer bzw. das Produkt herangefahren werden und für Produkte mit größerer Höhe muss der Abstand der Röntgenstrahlungsquelle erhöht werden.

Nachteilig bei dieser Vorrichtung ist jedoch, dass der obere Randbereich des zu untersuchenden Produkts immer von relativ schräg (in der Strahlebene) nach oben verlaufenden Teilstrahlen durchdrungen wird. Dies führt zu einer Verzerrung des betreffenden Bildberreichs, das durch die Abtastung erzeugt wird. Für bestimmte Anwendungsfälle ist es wünschenswert, dass auch ein oberer Bereich oder ein Bereich in einer vorbestimmten Höhe des zu untersuchenden Produkts von einem im Wesentlichen horizontal verlaufenden Teilstrahl durchdrungen wird, beispielsweise bei der Untersuchung des oberen Randes eines Glasgefäßes auf Beschädigungen, bei der Untersuchung des korrekten Sitzes eines Deckels oder beim Detektieren des Füllstandes (bzw. korrekten Füllstandes) eines Behälters.

Hierzu ist es möglich, die Röntgenstrahlungsquelle in der Höhe des oberen Randes des Produktes anzuordnen und den Strahlwinkel so zu definieren, dass auch noch der gesamte untere Bereich des Produkts vom Röntgenstrahl durchdrungen wird. Zur Anpassung an Produkte mit unterschiedlichen Höhen kann dann die Position der Röntgenstrahlungsquelle entsprechend vertikal eingestellt werden. Eine derartige rein vertikal bewegliche Röntgenquelle bedingt jedoch eine gleichzeitige Anpassung des Strahlwinkels, d.h. des Öffnungswinkels des fächerartigen Strahls, so, dass auch der untere Produktbereich (gerade noch) durchstrahlt wird. Dabei gilt es zu vermeiden, dass ein größerer Teil der Strahlungsenergie nicht durch das Produkt hindurchtritt (in der worst case-Ebene) oder ein interessierender Bereich des Produkts nicht durchstrahlt wird. Die Anpassung des Strahlwinkels bedingt jedoch einen zusätzlichen Aufwand.

Als Lösung wird in der US 7,970,102 B2 eine Vorrichtung zur Röntgenuntersuchung von bewegten Behältern beschrieben, bei der zwei Röntgenstrahlungsquellen und zwei zugehörige Detektorvorrichtungen verwendet werden. Eine erste Röntgenstrahlungsquelle ist seitlich neben dem Bandförderer in der Höhe des Förderbands bzw. der Unterseite des zu untersuchenden Behälters vorgesehen, wobei der untere Randstrahl des Röntgenstrahls im Wesentlichen horizontal verläuft und sich der Strahl in der Strahlebene mit einem vorbestimmten Strahlwinkel nach oben öffnet. Der Strahlwinkel kann so gewählt werden, dass ein oberer Teilbereich des Behälters nicht mehr von diesem Röntgenstrahl durchdrungen wird. Eine zweite Röntgenstrahlungsquelle ist seitlich neben dem Bandförderer in der Höhe des betreffenden Behälters angeordnet. Der von dieser Röntgenstrahlungsquelle erzeugte Strahl weist einen oberen Randstrahl auf, der im Wesentlichen horizontal verläuft. Der Strahl öffnet sich mit einem vorbestimmten Strahlwinkel in der Strahlebene nach unten. Der Strahlwinkel dieses Strahls kann so gewählt werden, dass ein unterer Teilbereich des Behälters nicht mehr durchdrungen wird. Dennoch ist bei dieser Variante sichergestellt, dass das gesamte Produkt, d.h. alle Teilbereiche des Behälters, zumindest unter Verwendung eines der beiden Röntgenstrahlen untersucht werden, der überwiegende (mittlere) Teil des Behälters sogar von beiden Röntgenstrahlen. Die zweite, obere Röntgenstrahlungsquelle kann auch vertikal verstellbar ausgebildet sein, um eine Anpassung an Behälter mit unterschiedlicher Höhe zu gewährleisten. Die Verstellbarkeit ist allerdings begrenzt, da sichergestellt werden muss, dass kein Teilbereich des Behälters von keinem der beiden Röntgenstrahlen durchdrungen wird.

Es handelt sich hierbei praktisch um zwei separate Scanvorrichtungen, die in Förderrichtung gesehen nacheinander angeordnet sein können. Sie können jedoch auch so angeordnet sein, dass die Behälter in unterschiedlichen Ebenen, die beispielsweise senkrecht aufeinander stehen, durchstrahlt werden.

Nachteilig bei dieser Vorrichtung ist allerdings der hohe Aufwand für die beiden Scanvorrichtungen.

Ausgehend von diesem Stand der Technik liegt der Erfindung die Aufgabe zugrunde, eine Vorrichtung zur Röntgenuntersuchung von bewegten Produkten, insbesondere bewegten Stückgütern zu schaffen, welche eine im Wesentlichen horizontale Durchstrahlung des zu untersuchenden Produkts im Bereich des oberen Randes bzw. in einer vordefinierten Höhe ermöglicht, bei welcher der Röntgenstrahl an Produkte mit unterschiedlicher Höhe anpassbar ist und welche mit möglichst geringem Aufwand realisierbar ist. Gleichzeitig soll die Vorrichtung die Durchstrahlung und damit Untersuchung eines Produkts in dessen Bodenbereich mit einem schräg zur Bewegungsebene des Produkts verlaufenden Teilstrahl ermöglichen.

Die Erfindung löst diese Aufgabe mit den Merkmalen des Oberbegriffs des Patentanspruchs 1.

Die Erfindung geht von der Erkenntnis aus, dass durch eine lineare Verschiebung der Röntgenstrahlungsquelle in einer Richtung parallel zur Richtung des unteren Randstrahls des von der Röntgenstrahlungsquelle erzeugten fächerartigen Röntgenstrahls eine einfache Anpassung an Produkte mit unterschiedlichen Höhen möglich ist. Dabei ist sichergestellt, dass der untere Randstrahl immer entlang derselben Geraden und schräg zur Bewegungsebene verläuft. Es ändert sich lediglich der Anfangspunkt des unteren Randstrahls, der beispielsweise durch den Austrittspunkt des fächerartigen Röntgenstrahls an der Röntgenstrahlungsquelle definiert sein kann. Bei einer Änderung der Position der Röntgenstrahlungsquelle entlang der Verschiebegeraden ändert sich selbstverständlich die Leistungsdichte der Röntgenstrahlung, mit welcher das Produkt bestrahlt wird. Die Bewegungsebene ist dabei definiert durch das Fördermittel bzw. die durch sie definierte Auflagefläche, in welcher sich die Unterseite bzw. die untersten Punkte des zu untersuchenden Produkts bewegen.

Die Gerade, entlang welcher die Verschiebung der Röntgenstrahlungsquelle und damit des fächerartigen Röntgenstrahls erfolgt, wird man üblicherweise so wählen, dass zumindest ein gewünschter unterer Bereich des zu untersuchenden Produkts durchstrahlt wird. Soll der gesamte untere Bereich eines Produkts durchstrahlt werden, so ist es in jedem Fall erforderlich, dass der untere Randstrahl auch durch die Auflagefläche bzw. die Unterseite des zu untersuchenden Produkts hindurchtritt, mit welcher dieses auf dem Fördermittel aufliegt.

Da die Richtung und Position des unteren Randstrahls bei der Verschiebebewegung der Röntgenstrahlungsquelle im Wesentlichen erhalten bleiben, ergibt sich der Vorteil, dass die Detektorvorrichtung bzw. der Sensor der Detektorvorrichtung, welcher in seiner Position fest ist, auch in dem Bereich, in welchem der untere durchstrahlte Bereich des Produkts abgebildet wird, optimal ausgenutzt wird. Die Höhe des Sensors der Detektorvorrichtung, welcher seitlich desFördermittels angeordnet ist, muss selbstverständlich so gewählt werden, dass der obere Randstrahl auch in der höchsten Positionen der Röntgenstrahlungsquelle noch auf den Sensor trifft.

An dieser Stelle sei erwähnt, dass es die Erfindung nicht zwingend erfordert, dass der obere Randstrahl horizontal verläuft. Vielmehr sind auch solche Ausführungsformen eingeschlossen, bei welchen der obere Randstrahl in einer beliebigen Richtung verläuft. Bei einer derartigen Ausführungsform kann beispielsweise der Füllstand eines Behälters mit hoher Genauigkeit erfasst werden, wenn der Pegel, d.h. die obere Grenzfläche des Mediums, mit welchem der Behälter gefüllt ist, einen größeren Abstand vom oberen Randbereich des Behälters aufweist. Die Position der Röntgenstrahlungsquelle wird man in diesem Fall so wählen, dass ein parallel verlaufender Teilstrahl des Röntgenstrahls in derjenigen Höhe verläuft, in welcher der zu erwartende Pegel des Füllmediums liegt. Bei einer derartigen Ausführungsform kann der oberhalb des Pegels liegende Teilbereich des Behälters, beispielsweise der Deckel oder Deckelbereich, gleichzeitig noch durch denjenigen Teilstrahl des fächerartigen Röntgenstrahls durchdrungen und damit inspiziert werden, der oberhalb des im Wesentlichen waagrecht verlaufenden Teilstrahls liegt.

Die Bewegung des zu untersuchenden Produkts zur Durchstrahlung kann dabei entlang einer Bewegungskurve erfolgen. Die Bewegungskurve kann selbstverständlich eine Gerade sein, d.h. die Produkte werden linear bewegt. Im allgemeinen Fall kann die Bewegung jedoch selbstverständlich entlang einer beliebig ausgebildeten Bewegungskurve erfolgen. Im Bereich der Bewegungskurse, in welchem die Durchstrahlung des zu untersuchenden Produkts erfolgt, wird die Bewegungskurve jedoch vorzugsweise in einer (beispielsweise horizontal liegenden) Ebene liegen.

Eine derartige Bewegung des zu untersuchenden Produkts ermöglicht die Abtastung des Produkts mit einem fächerartig ausgebildeten Röntgenstrahl, welcher senkrecht zur Strahlebene nur eine geringe Ausdehnung aufweist bzw. unter Verwendung eines Sensors, welcher in der Bewegungsrichtung nur eine geringe Ausdehnung besitzt, und insbesondere als Zeilensensor ausgebildet sein kann.

Die Bewegung des zu untersuchenden Produkts kann jedoch auch nur in einer Rotation des Produkts um eine Achse senkrecht zur Bewegungsebene, insbesondere um eine Längsachse oder Symmetrieachse des Produkts handeln, d.h. das Produkt wird im Bereich des fächerartigen Röntgenstrahls um eine Rotationsachse rotiert, so das der Röntgenstrahl das Produkt jeweils entlang einer anderen Schnittebene durchstrahlt.

Es ist auch möglich, die beiden vorgenannten Bewegungen zu kombinieren. Das Produkt kann entlang einer beliebigen Bewegungskurve in einen Bereich gefördert werden, in welchem die Durchstrahlung erfolgt. In dieser Position kann dann eine rein rotatorische Bewegung des zu untersuchenden Produkts selbst erfolgen, beispielsweise mittels eines rotierenden Tellers oder einer rotierenden Scheibe, deren Auflagefläche auch die Bewegungsebene definiert. Nach einem derartigen Scannen des Produkts kann das Produkt dann wieder entlang der vorgegebenen Bewegungskurve weitertransportiert werden.

Es ist jedoch auch möglich, die beiden Bewegungen gleichzeitig oder quasi gleichzeitig (beispielsweise in diskreten Schritten) auszuführen. Die Rotation des Produkts selbst um eine Rotationsachse kann gleichzeitig mit dem (Weiter-) Fördern des Produkts erfolgen.

Nach einer Ausführungsform der Erfindung kann die Röntgenstrahlungsquelle so ausgebildet und angeordnet sein, dass der Röntgenstrahl das Fördermittel in der gesamten Breite des Fördermittels durchdringt. Das Fördermittel muss in diesem Fall selbstverständlich so ausgestaltet sein, dass der untere Randstrahl bzw. derjenige Teilstrahl des fächerartigen Röntgenstrahls, welcher durch das Fördermittel hindurchtritt, noch ein brauchbares Bild auf dem Sensor der Detektorvorrichtung erzeugt. Das Fördermittel der Fördervorrichtung muss somit zumindest in dem Bereich, durch welchen der betreffende Teilstrahl hindurch tritt, aus einem geeigneten Material bestehen und/oder in geeigneter Weise aufgebaut sein. Da sich die Röntgenstrahlungsquelle auf einer Seite des Fördermittels der Fördervorrichtung und die Detektorvorrichtung bzw. der Sensor der Detektorvorrichtung auf der anderen Seite des Fördermittels und damit des zu untersuchenden Produkts befinden, verläuft der untere Randstrahl immer schräg (d.h. nicht senkrecht) zu der Fläche des Fördermittels, auf welchem das Produkt aufliegt. Die Fördervorrichtung kann demzufolge einen Teilbereich aufweisen , welcher nicht von dem fächerartigen Röntgenstrahl bestrahlt wird und eine stabilisierende Konstruktion oder eine Tragkonstruktion für das Fördermittel bildet.

Nach einer anderen Ausgestaltung der Erfindung kann die Röntgenstrahlungsquelle so ausgebildet und angeordnet sein, dass der fächerartige Röntgenstrahl das Fördermittel in einer Breite ausgehend von der der Detektorvorrichtung zugewandten Seite bis zu einem Randbereich an der gegenüberliegenden Seite durchdringt. Bei dieser Ausführungsform wird somit ein Teil des Fördermittels nicht bestrahlt. Damit kann dieser Teil des Fördermittels aus einem Material bestehen oder konstruktive Merkmale aufweisen, wodurch eine korrekte Abbildung behindert oder gar unmöglich gemacht würde.

Nach einer Ausgestaltung der Erfindung können die Fördervorrichtung ein Bandförderer und das Fördermittel ein Obertrum eines angetriebenen Endlosbandes sein. Derartige Bandförderer sind vielfältig verfügbar und stellen eine kostengünstige Alternative einer Fördervorrichtung dar. Grundsätzlich ist es jedoch ebenfalls möglich, beliebige andere Fördervorrichtungen zur Realisierung der Erfindung zu verwenden.

Nach einer Ausführungsform der Erfindung kann die Röntgenstrahlungsquelle so ausgebildet und angeordnet sein, dass der untere Randstrahl des fächerartigen Röntgenstrahls das Untertrum des Endlosbandes nicht durchdringt. Hierzu muss selbstverständlich der Bandförderer so ausgebildet sein, dass er eine Dicke bzw. Tiefe aufweist, die es ermöglicht, dass der untere Randstrahl den Querschnittsbereich des Bandförderers verlassen kann, bevor der untere Randstrahl das Untertrum des Endlosbandes des Bandförderers schneidet. Selbstverständlich sollte der Bandförderer in dem Querschnittsbereich, welcher von dem fächerartigen Röntgenstrahl durchstrahlt wird, so beschaffen sein, dass eine korrekte Abbildung ermöglicht wird.

Nach einer Ausgestaltung der Erfindung kann der Bandförderer einen Bandkörper aus einem röntgenstrahlungsreflektierenden und/oder stark absorbierenden Material umfassen, wobei ein oberes Trum des Endlosbandes auf einer Auflagefläche des Bandkörpers aufliegt. Der Bandkörper weist bei dieser Ausführungsform im Bereich des Röntgenstrahls eine einschnittartige Ausnehmung auf, durch welche der Röntgenstrahl verläuft. Der Röntgenstrahl durchstrahlt daher nur das Obertrum des Endlosbandes und die Ausnehmung. Die Ausnehmung kann in einfacher Weise so gestaltet werden, dass keine oder nur eine geringfügige Beeinträchtigung, insbesondere Dämpfung und/oder Streuung des Röntgenstrahls erfolgt.

Beispielsweise kann die Fördereinrichtung zumindest im Bereich der Auflagefläche mit einem Material gefüllt sein, welches die Röntgenstrahlung im Wesentlichen vollständig oder nur mit geringer Dämpfung transmittiert. Auf diese Weise kann sogar im Bereich des Einschnitts die Auflagefläche wieder geschlossen werden. Hierdurch kann auch die Stabilität verbessert werden.

Nach einer weiteren Ausführungsform der Erfindung kann zumindest ein Teilbereich der Außenwandung des Bandkörpers im Bereich der einschnittartigen Ausnehmung, welche nicht von der Ausnehmung betroffen ist, als Teil einerWandung eines Strahlenschutzgehäuses wirken. Dabei kann es sich insbesondere um einen Teil einer, bezogen auf die Förderrichtung der Bewegungskurve im Bereich der Durchstrahlung des Produkts axialen Umfangswandung des Strahlenschutzgehäuses wandeln. Der Einschnitt kann dazu beispielsweise an der Unterseite des Bandkörpers und/oder an derjenigen Seite, auf welcher die Röntgenstrahlungsquelle angeordnet ist, von einer Röntgenstrahlung absorbierenden und/oder reflektierenden Wandung begrenzt sein. Hierbei kann es sich um separate Wandungen oder um Außenwandungen des Bandkörpers handeln.

Nach einer Ausgestaltung der Erfindung kann der Bandkörper ein im Wesentlichen U-förmiges Profilstück durch Biegen eines röntgenstrahlungsreflektierenden und/oder stark absorbierenden, vorzugsweise im Wesentlichen ebenen, plattenförmigen Teils, beispielsweise eines Metallblechteils, hergestellt sein, welches eine die Ausnehmung für den röntgenstrahlbildenden Durchbruch aufweist. Auf diese Art und Weise kann der Bandkörper einfach und kostengünstig hergestellt werden. Insbesondere kann die Ausnehmung des Bandkörpers für den Röntgenstrahl bereits durch Stanzen oder Laserschneiden des plattenförmigen Teils hergestellt werden.

Das U-förmige Profilstück kann zwei Querwandungen aus einem röntgenstrahlungsreflektierenden und/oder stark absorbierenden Material, vorzugsweise zwei Blechwandungen, aufweisen. Dabei kann eine erste Querwandung, in Förderrichtung gesehen, vor der Ausnehmung und eine zweite Querwandung nach der Ausnehmung für den Röntgenstrahl vorgesehen sein. Zusätzlich kann eine Bodenwandung zusammen mit den beiden Querwandungen und jeweils vor und nach der Ausnehmung liegenden Bereichen der Dachwandung eine Strahlungsfalle bilden, welche zumindest einen wesentlichen Teil der Energie der Röntgenstrahlung des fächerartigen Strahls absorbiert, welche die Ausnehmung nicht auf einem im Wesentlichen geraden Weg durchdringt. Auf diese Weise kann insbesondere Streustrahlung, welche beim Durchtritt der Röntgenstrahlung durch das Obertrum des Endlosbandes entstehen kann, absorbiert werden.

Nach einer Ausführungsform der Erfindung kann der Bandkörper in seiner Auflagefläche eine in Förderrichtung verlaufende Nut aufweisen, welche in einem seitlichen Randbereich der Auflagefläche verläuft. Insbesondere kann es sich hierbei um den Randbereich der Auflagefläche handeln, welcher nicht mehr durch die Ausnehmung unterbrochen ist, wobei in die Nut ein Eingreifmittel des Endlosbandes, vorzugsweise eine in Förderrichtung verlaufende Erhebung an der Unterseite des Endlosbandes, eingreift, um Verschiebekräfte, die in einer Richtung quer zur Förderrichtung auf das Obertrum des Endlosbandes wirken, aufzunehmen, ohne dass das Obertrum quer zur Förderrichtung verschoben wird. Eine derartige Führung des Endlosbandes wird häufig erforderlich sein, da bei derartigen Vorrichtungen zur Röntgenuntersuchung von bewegten Produkten ein Strahlenschutzraum erforderlich ist, der in Richtung des ankommenden und abgehenden Produktstroms Öffnungen aufweist. Um das Austreten von Röntgenstrahlung aus diesen Öffnungen zu vermeiden, werden die Produkte innerhalb des Strahlenschutzraums gegenüber der Position, in welcher diese zugeführt werden, quer zur Förderrichtung versetzt. Ein derartiges Versetzen erfolgt meist mit Schiebern oder Schikanen, so dass auf das Endlosband Kräfte quer zur Förderrichtung wirken. Diese können ein Verschieben bzw. Verziehen des Endlosbandes bewirken, was durch die Nut und die Eingreifmittel vermieden wird.

Die Strahlungserzeugungsvorrichtung kann eine Linear-Verschiebevorrichtung aufweisen, welche für das lineare Verschieben der Röntgenstrahlungsquelle zusammen mit einer Hochspannungsquelle der Strahlungserzeugungsvorrichtung ausgebildet ist. Durch das gemeinsame Verschieben der eigentlichen Röntgenstrahlungsquelle mit der zugehörigen Hochspannungsquelle wird vermieden, dass ein geeignetes (d.h. entsprechend dickes) Kabel zwischen der bewegten Röntgenstrahlungsquelle und einer ortsfesten Hochspannungsquelle dauernd mitbewegt, insbesondere gebogen werden muss. Derartige Belastungen eines entsprechend dicken Hochspannungskabels können zu einer Beschädigung des Kabels führen. Um dies zu vermeiden, müsste ein entsprechend flexibles, meist teures Kabel verwendet werden.

In einer Ausgestaltung der Erfindung kann die Strahlungserzeugungsvorrichtung zusammen mit der Linear-Verschiebevorrichtung als Einheit ausgebildet sein. Die gesamte Einheit kann dabei so ausgestaltet sein, dass sie als Ganzes aus der Vorrichtung zur Röntgenuntersuchung demontierbar werden kann. Diese Einheit bzw. die Linear-Verschiebevorrichtung kann dabei auch den Antriebsmotor mit umfassen. Der Motor kann jedoch auch separat von der aus der Strahlungserzeugungsvorrichtung und der Linear-Verschiebevorrichtung bestehenden Einheit ausgebildet sein und bei einer Demontage der Einheit in der Vorrichtung zur Röntgenuntersuchen verbleiben.

Nach einer Ausführungsform der Erfindung kann die Röntgenstrahlungsquelle so ausgebildet sein, dass der obere Randstrahl des fächerartigen Röntgenstrahls horizontal verläuft, wobei die Auswerte- und Steuereinheit so ausgebildet ist, dass sie die Linear-Verschiebevorrichtung so steuert, dass der obere Randstrahl im Wesentlichen mit einer vorgegebenen Höhenposition, insbesondere der Höhe des jeweils zu untersuchenden Produkts, übereinstimmt.

Der fächerartige Röntgenstrahls kann jedoch auch einen horizontal verlaufenden Teilstrahl aufweisen, und die Auswerte- und Steuereinheit kann so ausgebildet sein, dass sie die Linear-Verschiebevorrichtung so ansteuert, dass der Teilstrahl im Wesentlichen mit einer vorgegebenen Höhenposition, insbesondere der Höhe, des jeweils zu untersuchenden Produkts übereinstimmt.

Weitere Ausführungsformen der Erfindung ergeben sich aus den Unteransprüchen.

Im Folgenden wird die Erfindung anhand eines in der Zeichnung dargestellten Ausführungsbeispiels näher erläutert. In der Zeichnung zeigen:
- Fig. 1: einen Schnitt durch eine Vorrichtung zur Röntgenuntersuchung von bewegten Produkten nach der Erfindung;
- Fig. 2: eine schematische Darstellung der wesentlichen Komponenten der Vorrichtung in Fig. 1 zur Erläuterung der Linearbewegung der Röntgenstrahlungsquelle;
- Fig. 3: eine perspektivische Ansicht eines Bandkörpers der für die Vorrichtung in Fig. 1 verwendeten Fördervorrichtung;
- Fig. 4: eine perspektivische Darstellung eines Blechteils zur Herstellung des Bandkörpers in Fig. 3; und
- Fig. 5: eine schematische Draufsicht auf eine weitere Ausführungsform einer Vorrichtung zur Röntgenuntersuchung von auf einer Kreisbahn bewegten Produkten.

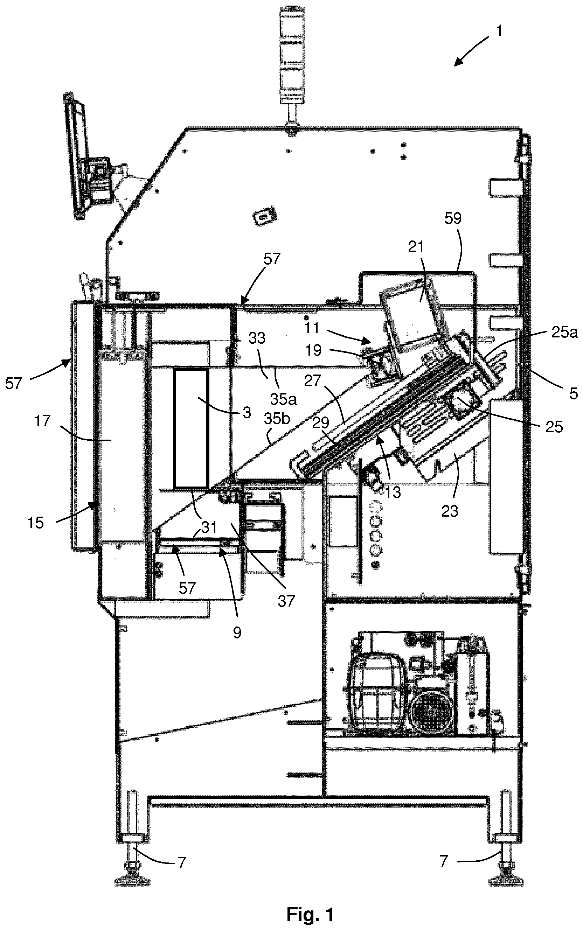

Die in Fig. 1 dargestellte Vorrichtung 1 zur Untersuchung von bewegten Produkten 3 ist als Standgerät ausgebildet und kann in einer Produktionsstraße (nicht dargestellt) verwendet werden. Sie kann jedoch selbstverständlich auch direkt in eine Produktionsstraße oder Produktionsmaschine integriert werden. Die Vorrichtung 1 weist ein Gehäuse 5 auf, welches an seiner Unterseite in der Höhe einstellbare Gerätefüße 7 besitzt. Die Vorrichtung 1 umfasst des Weiteren eine Fördervorrichtung 9, die im dargestellten Ausführungsbeispiel als Bandförderer ausgebildet ist. Grundsätzlich kann jedoch jede geeignete Fördervorrichtung verwendet werden, die es ermöglicht, zu untersuchende Produkte 3 entlang einer vorgegebenen Linie zu fördern. Das Fördern erfolgt vorzugsweise mit einer konstanten Geschwindigkeit. Es ist jedoch grundsätzlich ebenfalls möglich, die Produkte 3 schrittweise mit einer vorgegebenen Schrittweite zu bewegen und jeweils im Stillstand des betreffenden Produkts 3 einen vorzugsweise linienförmigen Teilbereich des Produkts 3 zu scannen. Grundsätzlich kann jedoch auch eine ungleichförmige Bewegung mit (positiven oder negativen Beschleunigungen) während des eigentlichen Scannens, d.h. der Datenaufnahmen, gegeben sein.

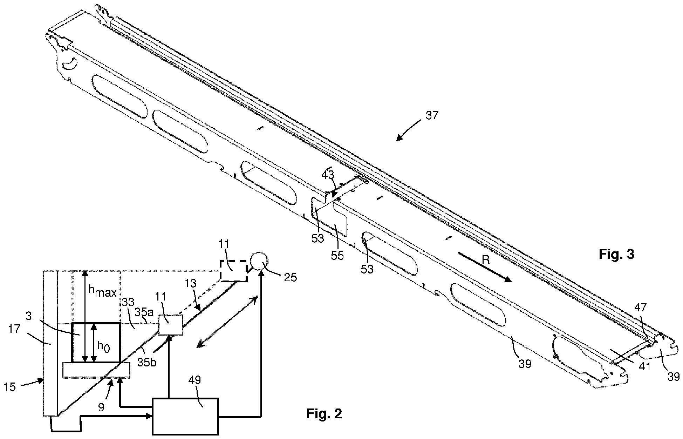

Der Bandförderer 9 transportiert die Produkte 3 durch das eigentliche Kernstück der Vorrichtung 1, welches gebildet ist durch eine linear bewegbare Röntgenstrahlungserzeugungsvorrichtung 11, eine Linear-Verschiebevorrichtung 13 und eine Detektorvorrichtung 15 zur Erfassung der Röntgenstrahlung, welche von der Röntgenstrahlungserzeugungsvorrichtung erzeugt wird und auf einen Sensor 17 der Detektorvorrichtung 15 trifft. Die Förderrichtung, die sich für die Bewegung der Produkte 3 ergibt, ist mit R bezeichnet (siehe Fig. 3).

Wie aus Fig. 1 ersichtlich, umfasst die Röntgenstrahlungserzeugungsvorrichtung 11 eine Röntgenstrahlungsquelle 19, welche die zur Erzeugung der Röntgenstrahlung erforderliche elektrische Energie von einer Hochspannungsquelle 21 bezieht. Die gesamte Röntgenstrahlungserzeugungsvorrichtung 11, also die Röntgenstrahlungsquelle 19 und die Hochspannungsquelle 21 wird als Modul mittels der Linear-Verschiebevorrichtung 13 entlang einer vorgegebenen Geraden bewegt. Auf diese Weise kann auf ein flexibles Spezialkabel mit großem Querschnitt zur elektrischen Energieversorgung der Röntgenstrahlungsquelle 19 verzichtet werden. Ein derartiges Kabel müsste ausreichend flexibel sein, um die fortwährende lineare Bewegung der Röntgenstrahlungsquelle zu ermöglichen. Derartige Kabel sind entsprechend teuer und werden zudem eine latente Fehlerquelle.

Die Linear-Verschiebevorrichtung 13 ist in einem Trägerrahmen 23 gehalten, welcher auf einfache Weise demontierbar im Gehäuse 5 gehalten ist. Damit kann die gesamte Einheit bestehend aus Röntgenstrahlungserzeugungsvorrichtung 11 und Linear-Verschiebevorrichtung 13 als Ganzes modular aus dem Gehäuse 5 demontiert werden. Dies kann zum Zweck des Austausches oder zu Wartungszwecken bzw. Reparaturzwecken erforderlich sein.

Die Linear-Verschiebevorrichtung 13 kann selbstverständlich einen Antrieb bekannter Art, beispielsweise einen Motor 25, insbesondere einen Elektromotor, aufweisen, welcher entweder direkt oder über ein Getriebe eine Gewindespindel 27 antreibt. Die Gewindespindel 27 kann ihrerseits einen linear geführten Schlitten 29 antreiben, auf welchem die Röntgenstrahlungserzeugungsvorrichtung 11 angeordnet ist.

Wie in Fig. 1 dargestellt, ist die Röntgenstrahlungserzeugungsvorrichtung 11 zusammen mit der Linear-Verschiebevorrichtung 13 so angeordnet, dass sich die Röntgenstrahlungsquelle 19 in einer Position seitlich neben dem zu untersuchenden Produkt bzw. seitlich neben dem Fördermittel befindet, welches das zu untersuchende Produkt bewegt. Im dargestellten Ausführungsbeispiel stellt ein Endlosband 31 des Bandförderers 9 das Fördermittel dar. Das Fördermittel kann im Allgemeinen jedoch jedes geeignete Mittel einer Fördervorrichtung sein, welches, ggf. angepasst an die zu fördernden Produkte, eine Bewegung der Produkte ermöglicht. Beispielsweise kann es sich auch um eine endlos umlaufende Förderkette oder eine vibrierende, leicht schräg verlaufende Platte handeln, auf welcher sich die Produkte gleitend bewegen. Das Fördermittel kann auch eine rein passive Gleitplatte sein, über welche das Produkt mit einer vorgegebenen Anfangsgeschwindigkeit hinweggleitet. Auch das Gleiten auf einem Luftpolster ist möglich.

Bei der Ausführungsform nach Fig. 1 ist die Röntgenstrahlungsquelle seitlich neben und oberhalb einer gedachten, horizontalen untersten Bewegungsebene angeordnet, in welcher die Unterseite bzw. der unterste Punkt der bewegten, zu untersuchenden Produkte liegt. Diese unterste Bewegungsebene wird im Folgenden auch als Bewegungsebene E bezeichnet. Sie wird bei vielen Ausführungsformen horizontal verlaufen, jedoch ist dies nicht zwingend erforderlich.

Die Bewegungsgerade, entlang welcher die Bewegung der Röntgenstrahlungsquelle 19 bzw. der gesamten Röntgenstrahlungserzeugungsvorrichtung 11 erfolgt, verläuft schräg zur Bewegungsebene E und beispielsweise senkrecht zur Förderrichtung R. Es ist jedoch ebenfalls möglich, die Röntgenstrahlungsquelle 19 bzw. die Röntgenstrahlungserzeugungsvorrichtung 11 schräg zur Förderrichtung R zu bewegen, solange die Bewegung eine Komponente senkrecht zur Förderrichtung R aufweist.

Wie aus Fig. 1 ersichtlich, erzeugt die Röntgenstrahlungsquelle 19 einen fächerartig ausgebildeten Röntgenstrahl 33 mit einem oberen Randstrahl 35a und einem unteren Randstrahl 35b. Die Randstrahlen stellen die gedachten Grenzen bzw. Ränder des einen bestimmten Öffnungswinkel aufweisenden Röntgenstrahls 33 dar und können jeweils als Strahl mit unendlich kleinem Öffnungswinkel betrachtet werden. Der fächerartige Röntgenstrahl 35 weist eine Strahlebene auf, welche vorzugsweise und auch im dargestellten Ausführungsbeispiel vertikal bzw. senkrecht zur Bewegungsebene Eder Produkte 3 liegt. Grundsätzlich ist es jedoch auch möglich, die Strahlebene schräg (aber nicht parallel) zur Bewegungsebeneverlaufen zu lassen und die Röntgenstrahlungsquelle 19 entsprechend auszubilden und/oder anzuordnen. Senkrecht zur Strahlebene weist der Röntgenstrahl 33 im dargestellten Ausführungsbeispiel nur eine relativ geringe Ausdehnung auf, da ein Sensor 17 in Form eines Zeilensensors verwendet wird. Dieser kann eine einzige Zeile aufweisen, welche für die Röntgenstrahlung empfindlich ist und beispielsweise - im Fall eines digitalen Sensors - eine Vielzahl von Pixeln aufweist. Es kann jedoch auch ein TDI-Zeilensensor verwendet werden, welcher mehrere Zeilen aufweist, deren Signale, angepasst an die (konstante) Bewegungsgeschwindigkeit der Produkte in der Richtung, von einer Zeile zur nächsten weiterverschoben und so integriert werden. Auch bei einem TDI-Sensor wird jedoch als Ausgangssignal jeweils das Abbild einer Zeile bzw. einer entsprechenden "Scheibe" des durchstrahlten Produkts geliefert. Der Begriff Sensor oder TDI-Sensor soll dabei alle die Röntgenstrahlung direkt wandelnden Bauelemente umfassen und auch solche Bauelemente, bei denen die Röntgenstrahlung erst in ein anderen spektralen Bereich umgesetzt wird, beispielsweise über Szintillatoren.

Die Richtung des Zeilensensors 17 muss selbstverständlich so gewählt werden, dass diese mit der Strahlebene fluchtet. Der fächerartige Röntgenstrahl 33 muss nach dem Durchdringen des Produkts 3 auf den empfindlichen Bereich des Zeilensensors 17 treffen. Weist der Röntgenstrahl 33 in der Richtung quer zur Strahlebene eine größere Ausdehnung auf als die empfindliche Fläche des Zeilensensors 17, so "schneidet" der Zeilensensor 17 aus dem Röntgenstrahl 33 ein Abbild aus, welches einer "Scheibe" des durchstrahlten Produkts 3 mit entsprechender Dicke entspricht.

Grundsätzlich ist es ebenfalls möglich, anstelle eines Zeilensensors einen zweidimensional empfindlichen Sensor 17 (Flächensensor) zu verwenden. Dieser kann entweder einen, in Förderrichtung R gesehen, entsprechend größeren Abschnitt des Produkts 3 abbilden oder sogar das gesamte Produkt. Voraussetzung hierfür ist selbstverständlich ein entsprechend angepasster, breiterer Röntgenstrahl.

Die Röntgenstrahlungsquelle 19 und die Linear-Verschiebevorrichtung 13 sind so ausgebildet und angeordnet, dass der untere Randstrahl 35b in seiner Position und Richtung auch bei der linearen Bewegung der Röntgenstrahlungsquelle 19 unverändert entlang derselben Geraden verläuft. Es ändert sich lediglich der Ausgangspunkt des unteren Randstrahls 35b. Dies hat zur Folge, dass der untere Randstrahl 35b immer in im Wesentlichen demselben Punkt bzw. in derselben Höhe auf einen unteren Bereich des Sensors 17 trifft. Dies kann bevorzugt auch der unterste empfindliche Punkt bzw. der unterste empfindliche Bereich des Sensors 17 sein. Es wird hierdurch erreicht, dass auch bei einer Verschiebung der Röntgenstrahlungsquelle dieser unterste bestrahlte Bereich des Sensors 17 im Wesentlichen unverändert bleibt. Bei einer Verschiebung ändert sich lediglich der Auftreffpunkt des oberen Randstrahls 35a. Die Abbildung des zu untersuchen Produkts 3 erfolgt also immer so, dass der untere bzw. der unterste durchstrahlte Bereich des Produkts 3 von einem zugeordneten unteren bzw. untersten Bereich des Sensors 17 erfasst wird.

Üblicherweise wird man den unteren Randstrahl 35b in Bezug auf die Position des zu untersuchenden Produkts 3 und die Ausbildung der Fördervorrichtung 9 so wählen, dass das gesamte Produkt bzw. der gesamte untere Bereich des Produkts 3 durchstrahl wird. Es ist jedoch selbstverständlich ebenfalls möglich, den unteren Randstrahl 35b so festzulegen, dass ein vorgegebener unterer Teilbereich des Produkts 3 nicht durchstrahlt wird, beispielsweise wenn dieser Bereich in Bezug auf die vorzunehmende Prüfung des Produkts unkritisch bzw. uninteressant ist.

Falls dies gewünscht sein sollte, kann der untere Randstrahl auch schräg nach oben verlaufen, wozu die Röntgenstrahlungsquelle 19 bzw. die gesamte Röntgenstrahlungserzeugungsvorrichtung 11 und die Linear-Verschiebevorrichtung 9 selbstverständlich seitlich unterhalb der Bewegungsebeneder Produkte 3 positioniert werden muss. Der obere Randstrahl 35a verläuft in diesem Fall steiler (nach oben) als der untere Randstrahl 35b. Auch in diesem Fall wird der Sensor in seinem unteren Bereich immer vollständig ausgenutzt, unabhängig von der Höhe der zu untersuchenden Produkte bzw. unabhängig von der Position der Röntgenstrahlungsquelle 19.

Auch bei dieser Ausführungsform wird der Vorteil erreicht, dass zur Detektion bestimmter Fehlerquellen der Bodenbereich des zu untersuchenden Produkts schräg durchstrahlt wird. Beispielsweise lassen sich so Glassplitter, die sich im Bodenbereich des Behältervolumens befinden, auch bei gewölbten Böden, die beispielsweise eine zentrale Einbuchtung aufweisen, sicher detektieren. Denn die schräg verlaufend Teilstrahlen treten maximal zweimal durch die Behälterwandungen (incl. Boden des Behälters) hindurch, während ein waagrecht verlaufenden Teilstrahl vielfach durch Behälterwandungen hindurchträte und dadurch gleichartige Fremdkörper schwer oder überhaupt nicht mehr detektierbar wären.

Bei der in Fig. 1 dargestellten Ausführungsform einer Vorrichtung 1 ist die Röntgenstrahlungserzeugungsvorrichtung 11 bzw. die Röntgenstrahlungsquelle 19 der Vorrichtung 1 zur Röntgenuntersuchung von bewegten Produkten so ausgebildet und angeordnet, dass der obere Randstrahl horizontal bzw. parallel zur Bewegungsebeneverläuft. Hierdurch ergibt sich der Vorteil, dass bestimmte Eigenschaften eines Produkts in der Höhe des horizontal verlaufenden oberen Randstrahls 35a mit guter Genauigkeit oder überhaupt erst mit der gewünschten Sicherheit erfasst werden können. Beispielsweise kann der Füllstand eines mit einem Medium gefüllten Gefäßes mit höherer Genauigkeit detektiert werden, wenn der Pegel in etwa parallel zum Verlauf des betreffenden Teilstrahls bzw. des oberen Randstrahls 35a verläuft. Auch die Untersuchung des oberen Randes eines Produkts bzw. Gefäßes, wie beispielsweise der obere Rand eines Glasgefäßes, kann auf diese Art und Weise vorteilhaft überprüft werden, beispielsweise auf das Vorliegen von Beschädigungen.

Insbesondere ist es auf diese Weise möglich, die Unversehrtheit des Gewindes einer Glasflasche bzw. eines Glasbehälters zu kontrollieren, beispielsweise auch in bereits verschraubtem Zustand. Denn bei solchen Behältern kann das Glasgewinde durch das Aufschrauben des Deckels bereits so vorgeschädigt sein, dass beim Öffnen des Deckels durch den Verbraucher das abgeplatzte Glasstück in den Behälter hineinfällt und das Lebensmittel erst dann kontaminiert. Das Abplatzen von Glassplittern am Deckelgewinde derartiger Behälter kann durch Glastoleranzen verursacht werden. Insbesondere beim automatischen Aufsetzen bzw. Aufschrauben des Deckels kann es dann in ungünstigen Fällen zum Abplatzen von Glassplittern im Gewindebereich kommen. Durch die vorbeugende präzise Vermessung des Deckelbereichs durch eine Röntgenuntersuchung können nach dem Aufschrauben von Deckeln bei derartigen Behältern solche Fehler festgestellt werden. Die fehlerhaften Produkte bzw. Behälter können dann vor dem Inverkehrbringen aussortiert werden.

Grundsätzlich muss nicht der obere Randstrahl 35a verwendet werden, um einen bestimmten Bereich eines Produkts unter Verwendung eines horizontal verlaufenden Teilstrahls zu untersuchen. Dies kann bei einem fächerartig ausgebildeten Röntgenstrahl 33, welcher einen horizontalen Teilstrahl mit umfasst, auch in der Weise erfolgen, dass die Vorrichtung 1 abhängig von der Position der Röntgenstrahlungsquelle 19 die Position bzw. Höhe kennt, in welcher der betreffende horizontale Teilstrahl auf den Sensor 17 der Detektorvorrichtung 15 trifft.

Bei der in Fig. 1 dargestellten Vorrichtung 1 ist der im Schnitt dargestellte Bandförderer 9 nur vereinfacht dargestellt. Das Endlosband 31 läuft um einen Bandkörper 37 und wird von einem nicht näher dargestellten Motor angetrieben. Der Bandkörper 37 ist in Fig. 3 in einer perspektivischen Ansicht dargestellt. Der Bandkörper 37 weist ein im Querschnitt im Wesentlichen U-förmiges Profil auf, welches zwei Schenkelwandlungen 39 und eine Dachwandung 41 aufweist. Das Endlosband 31 wird von einem nicht näher dargestellten Motor, insbesondere einem Elektromotor, angetrieben. Das Endlosband 31 ist des Weiteren um zwei ebenfalls nicht näher dargestellte Umlenkrollen geführt, die jeweils am Ende des Bandkörpers 37 angeordnet und rotierbar gelagert sind. Eine der beiden Endlosrollen kann durch den Elektromotor angetrieben sein. Es kann ein Spannmechanismus vorgesehen sein, der beispielsweise eine der Umlenkrollen mit einer Federkraft beaufschlagt, so dass insbesondere das obere Trum des Endlosbandes 31 mit einer vorgegebenen Spannkraft beaufschlagt wird. Das obere Trum des Endlosbandes 31 liegt auf der Oberseite der Dachwandung 41 auf.

In dem Bereich, in welchem der fächerartige Röntgenstrahl 33 die Fördervorrichtung 9 und damit auch den Querschnitt des Bandkörpers 37 schneidet, weist der Bandkörper 37 eine einschnittartige Ausnehmung 43 auf. Die Breite der Ausnehmung (in Längsrichtung des Bandkörpers 37 gesehen) ist zumindest so groß, dass derjenige Teil der Strahlungsenergie des fächerartigen Röntgenstrahls 33 ungehindert durch den Bandkörper hindurchtreten kann, welcher auch auf den Sensor 17 der Detektorvorrichtung 15 auftrifft. Hierdurch wird vermieden, dass der Röntgenstrahl 33, beim Durchlaufen des Querschnitts des Bandkörpers 37 (insbesondere ungleichmäßig) durch das Material des Bandkörpers 37 absorbiert (das heißt gedämpft) und/oder reflektiert wird. Eine derartige Ausnehmung 43 im Bandkörper 37 ist insbesondere dann erforderlich, wenn der Bandkörper, wie häufig üblich, aus einem für Röntgenstrahlung undurchdringlichen bzw. in hohem Maß absorbierenden und/oder reflektierenden Material besteht, wie beispielsweise einem Metall.

Bei einem Bandkörper 37 mit dem in Fig. 3 dargestellten Aufbau ist die einschnittartige Ausnehmung 43 insbesondere in einem Teil der Schenkelwandung 39 und der Dachwandung 41 vorgesehen. Der Einschnitt in der Dachwandung 41 des Bandkörpers 37 kann auch mit einem die Röntgenstrahlung nicht oder nur schwach dämpfenden und/oder schwach reflektierenden Material, wie beispielsweise einem Kunststoff (z.B. PEEK), geschlossen sein. Es kann auch die gesamte Ausnehmung 43 mit einem die Röntgenstrahlung nicht oder nur schwach dämpfenden und/oder schwach reflektierenden Material ausgefüllt sein.

Wie aus Fig. 3 ersichtlich, kann, in Förderrichtung R gesehen, vor und nach der Ausnehmung 43 jeweils eine vertikal verlaufende Querwandung 53 vorgesehen sein, welche zusammen mit einer Bodenwandung 55 eine Strahlungsfalle für denjenigen Teil der Röntgenstrahlung bildet, welche die Ausnehmung 43 nicht auf geradem Wege durchtritt. Beispielsweise kann Röntgenstrahlung, deren Richtung durch das durchstrahlte Material bzw. das durchstrahlte Produkt 3 und/oder durch das Material des durchstrahlten Fördermittels oder Endlosbandes 9 verändert wird (insbesondere Streustrahlung oder reflektierte Strahlung) und in die Strahlungsfalle eintritt, durch Mehrfachreflektion innerhalb der Strahlungsfalle so weit absorbiert werden, dass beim Austreten dieser Strahlung deren Leistung für Mensch und Maschine nicht mehr gefährlich ist. Die Querwandung 53 und die Bodenwandung 55 können dabei aus einem röntgenstrahlungsreflektierenden und/oder zumindest zum Teil absorbierenden Material bestehen. Stattdessen oder zusätzlich können die Innenseiten dieser Wandlungen auch mit einer entsprechenden Schicht aus einem geeigneten Material versehen sein. An seiner Oberseite ist die Strahlungsfalle durch diejenigen (horizontal verlaufenden) Bereiche der Dachwandung 41 begrenzt, in welchen nicht die Ausnehmung 43 bzw. deren Teilbereich in der Dachwandung 41 verläuft.

Die Bodenwandung 55 dieser Strahlungsfalle kann auch, wie aus Fig. 1 ersichtlich, einen Teil einer Strahlenschutzwandung oder eines Strahlenschutzgehäuses 57 bilden. Das Strahlenschutzgehäuse 57 ist allseitig fast vollständig geschlossen und weist nur eine Eintrittsöffnung und eine Austrittsöffnung für das Zuführen bzw. Abführen der Produkte 3 in den bzw. aus dem Strahlenschutzgehäuse 57 auf. In Förderrichtung R gesehen kann das Strahlenschutzgehäuse im Wesentlichen auf die Breite des Röntgenstrahls 33 in dieser Dimension begrenzt sein. Aus Sicherheitsgründen wird man das Strahlenschutzgehäuse in dieser Richtung jedoch auch mit einer größeren Breite versehen.

Wie aus Fig. 1 ersichtlich, kann das Strahlenschutzgehäuse 57 einen demontierbaren Deckel oder ein demontierbares Gehäuseteil 59 aufweisen, welches dazu dient, im Wartungsfall oder Reparaturfall die Röntgenstrahlungserzeugungsvorrichtung 11 aus dem Strahlenschutzgehäuse 57 auszubauen. Hierzu kann in vorteilhafter Weise die Röntgenstrahlungserzeugungsvorrichtung 11 mit der Röntgenstrahlungsquelle 19 und der Hochspannungsquelle 21 zusammen mit der Linear-Verschiebevorrichtung 13 als Einheit ausgebildet sein. Diese gesamte Einheit kann dann durch das Lösen einer geeigneten Befestigung, beispielsweise durch das Lösen einer oder mehrerer Schrauben, als Ganzes aus dem Strahlenschutzgehäuse 57 entnommen werden. Die Befestigung kann auch als werkzeuglos betätigbare Schnellspannvorrichtung ausgeführt sein. Hierdurch werden Wartung, Reparatur oder Austausch deutlich vereinfacht.

Bei der in Fig. 1 dargestellten Variante sind der Motor 25 und der Trägerrahmen 23 außerhalb des Strahlenschutzgehäuses 57 angeordnet und verbleiben beim Ausbau des Moduls, welches die Röntgenstrahlungserzeugungsvorrichtung 11 mit der Röntgenstrahlungsquelle 19 und der Hochspannungsquelle 21 sowie den Schlitten 29 umfasst, in der Vorrichtung 1. Zum Ausbau des Moduls muss lediglich der Antrieb vom Schlitten 29 abgekoppelt werden. Hierzu wird ein durch das Strahlenschutzgehäuse 57 geführter Antriebsriemen 25a, beispielsweise ein Zahnriemen, vom betreffenden Antriebselement des Schlittens 29, nämlich der Spindel 27, getrennt.

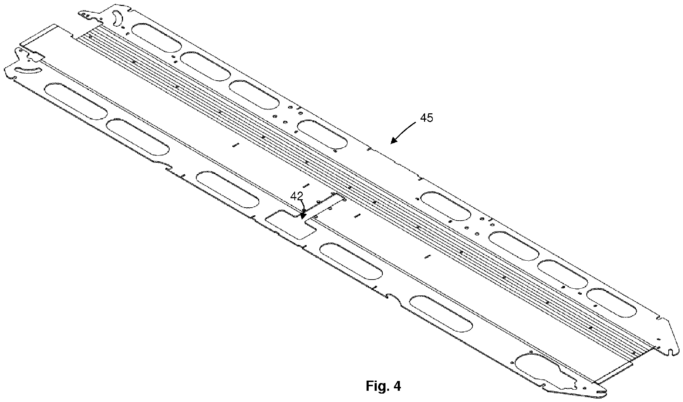

Wie aus Fig. 4 ersichtlich, wird der in Fig. 3 dargestellte Bandkörper 37 vorzugsweise aus einem ebenen, plattenförmigen vorgefertigten Element 45, beispielsweise einem Metallblech, hergestellt. Das Element 45 wird vorzugsweise in ebenem Zustand mit den erforderlichen Ausnehmungen oder Durchbrüchen hergestellt. Dabei kann es sich insbesondere um Durchbrüche zur Aufnahme von Achsen oder Lagern für die Umlenkrollen (nicht dargestellt) handeln und um einen Durchbruch 42, welcher nach dem Biegen die Ausnehmung 43 für den Röntgenstrahl 33 bildet.

Aus Fig. 3 ist des Weiteren ersichtlich, dass in demjenigen Randbereich der Oberseite, d.h. der Dachwandung 41 des Bandkörpers 37, welcher nicht durch den Röntgenstrahl 33 durchstrahlt wird, eine in Bewegungsrichtung des Endlosbandes 31 verlaufende Nut 47 vorgesehen ist. Zur Vereinfachung der Darstellung sind in Fig. 3 nur die Biegekanten der Nut 47 eingezeichnet. Diese Biegekanten zeigt auch Fig. 4.

Abschließend soll die Funktion der Vorrichtung 1 zur Röntgenuntersuchung von bewegten Produkten 3 anhand der schematischen Darstellung in Fig. 2 erläutert werden. Fig. 2 zeigt in einer ersten, mit durchgezogenen Linien dargestellten Position die Röntgenstrahlungserzeugungsvorrichtung 11 in einer untersten Position. In dieser Position können insbesondere Produkte 3 mit einer minimalen Höhe h₀ untersucht werden. Der obere Randstrahl 35a verläuft dabei im Wesentlichen (horizontal) fluchtend mit einer (zur Bewegungsebene E parallelen) obersten Bewegungsebene des Produkts 3 (in welcher der höchste Punkt des Produkts 3 bewegt wird). Es kann somit der oberste Bereich des Produkts 3 mit der Höhe h₀ mit einem horizontal verlaufenden Teilstrahl (nämlich dem obersten Randstrahl 35a) untersucht werden.

In einer zweiten, obersten Position ist die Röntgenstrahlungserzeugungsvorrichtung 11 mit gestrichelten Linien gezeigt. In dieser obersten Position können Produkte 3 mit einer maximalen Höhe hₘₐₓ untersucht werden, wobei auch in dieser Position der obere Randstrahl 35a horizontal fluchtend mit der obersten Bewegungsebene verläuft. Die Bewegung der Röntgenstrahlungserzeugungsvorrichtung 11 erfolgt zwischen der obersten und der untersten Position entlang einer Bewegungsrichtung parallel zur Richtung des unteren Randstrahls 35b.

Wie bereits vorstehend erläutert, trifft der untere Randstrahl 35b in jeder möglichen Position zwischen den beiden in Fig. 2 dargestellten Extrempositionen in demselben Punkt bzw. derselben Position auf den Sensor 17 der Detektorvorrichtung 15. Es variiert lediglich die Höhe, in welcher der obere Randstrahl 35a auf den Sensor 17 trifft.

Dabei ist es nicht zwingend erforderlich, dass die Höhe des oberen Randstrahls 35a mit der Höhe des jeweils zu untersuchenden Produkts 3 fluchtet bzw. abschließt (oder geringfügig darüber hinausragt). Die Position der Röntgenstrahlungserzeugungsvorrichtung 11 kann auch so eingestellt werden, dass der obere Randstrahl mit einer vordefinierten Höhe fluchtet, in welcher das Produkts 3 mit dem horizontal verlaufenden oberen Randstrahl 35a untersucht werden soll. Hierbei kann es sich um den Füllstand des betreffenden Produkts (Behälters) handeln.

Die Steuerung der Position der Röntgenstrahlungserzeugungsvorrichtung 11 bzw. der Röntgenstrahlungsquelle 19 und die Ansteuerung der Fördervorrichtung bzw. des Bandförderers 9 kann eine Auswerte- und Steuereinrichtung 49 übernehmen. Zur Einstellung der Position der Röntgenstrahlungserzeugungsvorrichtung 11 steuert die Auswerte- und Steuervorrichtung 49 den Motor 25 der Linear-Verschiebevorrichtung 13 an. Den Röntgenstrahl 33 kann die Auswerte- und Steuervorrichtung 49 durch die Ansteuerung der Röntgenstrahlungserzeugungsvorrichtung 11 selbst aktivieren und deaktivieren sowie in seiner Leistung beeinflussen. Die Auswerte- und Steuervorrichtung 49 kann auch so ausgebildet sein, dass sie die Fördervorrichtung bzw. den Bandförderer 9 hinsichtlich seiner Geschwindigkeit ansteuert. Dabei kann es sich auch um eine Ansteuerung im Sinne eines Bewegungsprofils handeln. Der Antrieb bzw. der Motor 25 kann hierzu auch einen Encoder aufweisen, um eine exakte Ansteuerung von Positionen innerhalb des Bewegungsprofils zu ermöglichen. Von der Detektorvorrichtung 15 erhält die Auswerte- und Steuervorrichtung 49 ein Signal, welches die vom Sensor 17 erfasste Bildinformation beinhaltet.

Auf diese Weise ist es der Auswerte- und Steuereinheit 49 möglich, die Position der Röntgenstrahlungserzeugungsvorrichtung 11 an die Höhe der zu untersuchenden Produkte 3 anzupassen. Dies kann entweder dadurch erfolgen, dass der Auswerte-und Steuervorrichtung 49 von einer übergeordneten Steuereinheit (nicht dargestellt) entsprechende Informationen zugeführt werden.

Die Auswerte- und Steuereinheit 49 kann jedoch auch so ausgebildet sein, dass sie eine automatische Adaption der Position der Röntgenstrahlungsquelle 19 an die Höhe des zu erfassenden Produkts 3 oder die zu erfassende Füllstandshöhe durchführt. Hierzu kann die Auswerte- und Steuereinheit 49 das zu erfassende Produkt zunächst in der obersten Position der Röntgenstrahlungserzeugungsvorrichtung 11 erfassen bzw. untersuchen und dann aus dem auf diese Weise erzeugten Bild die Höhe des betreffenden Produkts 3 ermitteln. Das erfasste Produkt 3 kann dann durch eine entsprechende Ansteuerung der Fördervorrichtung 9 wieder zurückbewegt und nach einer Positionierung der Röntgenstrahlungserzeugungsvorrichtung 11 in die gewünschte Position erneut vorwärts bewegt und untersucht werden. Die Röntgenstrahlungserzeugungsvorrichtung 11 kann dann so lange in der auf diese Weise ermittelten Position belassen werden, bis die Auswerte-und Steuereinheit 49 anhand des aktuell erfassten Bildes eines Produkts 3 feststellt, dass das obere Ende des Produkts 3 nicht mehr vom Bild erfasst wird. Diese Feststellung kann dann getroffen werden, wenn die Position der Röntgenstrahlungserzeugungsvorrichtung 11 immer so gewählt wird, dass der Röntgenstrahl 33 mit seinem oberen Randstrahl 35a die volle Erfassung der gesamten Produkthöhe ermöglicht. Hierzu muss die Position der Röntgenstrahlungserzeugungsvorrichtung 11 so gewählt werden, dass der obere Randstrahl 35a geringfügig höher liegt als die maximale Höhe des betreffenden zu untersuchenden Produkts 3.

Die Erfindung gewährleistet somit die einfache Anpassung des mittels der Röntgenstrahlungsquelle 19 erzeugten fächerartigen Röntgenstrahls an die Höhe eines zu untersuchenden Produkts 3. Durch das Verschieben der Röntgenstrahlungserzeugungsvorrichtung 11 bzw. der Röntgenstrahlungsquelle 19 in einer Richtung parallel zum unteren Randstrahl 35b des fächerartigen Röntgenstrahls 33 ergibt sich der Vorteil, dass der untere Randstrahl unabhängig von der Produkthöhe immer in derselben Position auf den Sensor 17 trifft. Die Position des unteren Randstrahls 35a kann so gewählt werden, dass gerade der gesamte untere Bereich des zu untersuchenden Produkts 3 noch voll auf dem Sensor 17 abgebildet wird. Selbstverständlich ist es ebenfalls möglich, den unteren Randstrahl 35a so festzulegen, dass ein bestimmter, nicht interessierender unterer Bereich des Produkts 3 nicht erfasst wird.

Ein oberer, horizontal verlaufender Randstrahl 35a oder ein (im Bereich des fächerartigen Röntgenstrahlen 33 liegender) horizontal verlaufender Teilstrahl kann durch eine einfache Positionierung der Röntgenstrahlungserzeugungsvorrichtung 11 so gewählt werden, dass sich diese in einer gewünschten Höhe des Produkts befindet, insbesondere in der Höhe eines Füllpegels oder im Bereich des oberen Produktrandes, in dem sich ein Behälterdeckel befinden kann.

Wird die Röntgenstrahlungserzeugungsvorrichtung 11 so positioniert, dass der erzeugte Röntgenstrahl nicht mehr als die zu untersuchende Höhe des Produkts 3 durchstrahlt, so kann die für die Untersuchung erforderliche Röntgenleistung minimiert werden. Die Auswerte- und Steuervorrichtung 49 kann dabei die Röntgenstrahlungsquelle 19 so ansteuern, dass die Bestrahlungsstärke der Röntgenstrahlung konstant bleibt. Bei der Untersuchung von Produkten 3 mit geringerer Höhe kann die Leistung der erzeugten Röntgenstrahlung, die der fächerartige Röntgenstrahl 33 transportiert, somit geringer sein als bei der Bestrahlung von Produkten 3 mit größerer Höhe bzw. bei der Durchstrahlung eines größeren Bereichs eines Produkts 3. Durch die Reduzierung der Röntgenstrahlungsleistung kann die Lebensdauer der Röntgenstrahlungsquellen erhöht werden.

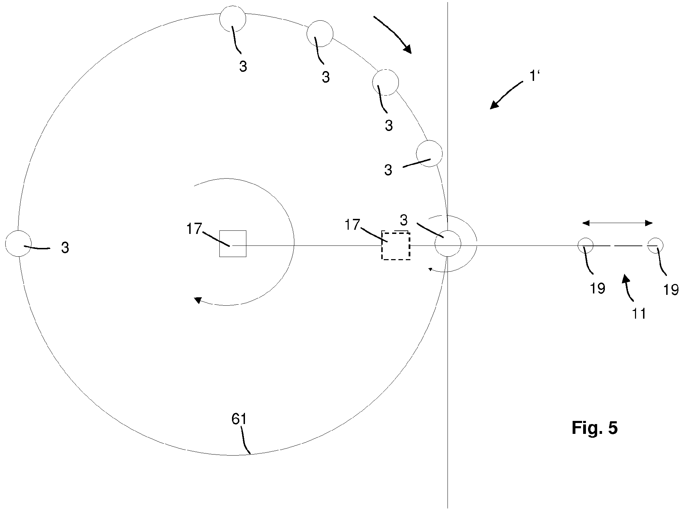

In Fig. 5 ist eine weitere Ausführungsform einer Vorrichtung 1' zur Röntgenuntersuchung von bewegten Produkten 3 in einer schematischen Draufsicht dargestellt. Die Produkte 3 werden entlang einer Bewegungskurve 61 bewegt. Die Bewegungskurve 61 ist im dargestellten Ausführungsbeispiel als Kreisbahn ausgeführt. Im Zentrum der Bewegungskurve 61 ist der Sensor 17 positioniert. Die Erfassungsrichtung des Sensors 17 weist radial nach außen. In Fig. 5 ist ein zu untersuchendes Produkt 3 in einer Erfassungsposition dargestellt. Die Röntgenstrahlungserzeugungsvorrichtung 11, die selbstverständlich wieder eine Röntgenstrahlungsquelle 19 und eine Hochspannungsquelle 21 aufweist, die mittels einer in Fig. 5 nicht näher dargestellten Linear-Verschiebevorrichtung derart verschiebbar ausgebildet ist, wie dies in Bezug auf die Fig. 1 bis 4 beschrieben wurde, ist an einer in Bezug auf die Bewegungskurve 61 dem Sensor 17 gegenüberliegenden Positionen vorgesehen. Die grundsätzlichen Funktionalitäten von Sensor 17 und Röntgenstrahlungserzeugungsvorrichtung 11 können identisch ausgebildet sein, wie dies im Zusammenhang mit den Fig. 1 bis 4 vorstehend erläutert wurde.

Wie dies in Fig. 5 gestrichelt angedeutet ist, können der Sensor 17 oder auch die gesamte Detektorvorrichtung 15 jedoch auch nicht im Zentrum der kreisförmigen Bewegungskurve 61 vorgesehen sein, sondern in einer Position, welche näher an die Erfassungsposition herangerückt ist.

Die Untersuchung des Produkts kann mit einer so ausgebildeten Vorrichtung in gleicher Weise erfolgen, wie dies zuvor in Verbindung mit den Fig. 1 bis 4 erläutert wurde, nämlich durch eine kontinuierliche oder schrittweise Bewegung entlang der Bewegungskurve, welche bei dieser Ausführungsform im Erfassungsbereich nicht mehr rein linear verläuft. Selbstverständlich kann das Produkt 3 dabei zeilenweise oder schrittweise abgetastet oder, bei Verwendung eines geeigneten Flächensensors, auch durch eine einzige Durchstrahlung bzw. Aufnahme untersucht werden.

Wie in Fig. 3 angedeutet, kann das Produkt 3 in seiner Erfassungsposition bzw. im Erfassungsbereich mit dem in Fig. 3 nicht näher dargestellten Fördermittel nach dem Erreichen seiner Erfassungsposition nur noch um eine Achse, vorzugsweise eine längs-oder Hochachse des Produkts 3 rotiert werden. Das Fördermittel kann hierzu im Erfassungsbereich als Drehteller oder Drehscheibe ausgebildet sein, wobei die Oberseite der Drehscheibe die Bewegungsebene E bildet. Die Rotationsbewegung des Produkts 3 erfolgt dann derart kontinuierlich oder schrittweise, dass das zu untersuchende Produkt in einer jeweils anderen Ebene durchstrahlt bzw. untersucht wird.

Es ist selbstverständlich ebenfalls möglich, die beiden vorgenannten Bewegungen zu kombinieren, d.h. das zu untersuchende Produkt 3 wird sowohl entlang der vorgegebenen Bewegungskurve 61 transportiert als auch um eine Rotationsachse rotiert. Dies setzt selbstverständlich ein etwas komplexer ausgebildetes Fördermittel voraus.

Auch in diesem Fall muss das Fördermittel so ausgebildet sein, dass der schräg zur Bewegungsebene E verlaufende untere Randstrahl 35b bzw. derjenige Teil des fächerartigen Röntgenstrahls 35 durch das Bewegungsmittel zumindest mit einer ausreichend geringen Dämpfung und Streuung hindurch treten kann, um auch eine zuverlässige Untersuchung des Produkts 3 in seinem unteren Bereich zu gewährleisten.

Selbstverständlich wäre es auch möglich, auf eine Bewegung des Produkts 3 entlang einer vorgegebenen Bewegungskurve zu verzichten und das zu untersuchende Produkt 3 manuell oder mittels eines Roboters oder einer geeigneten Vorrichtung in den Erfassungsbereich zu bringen, in welchem dann eine rotatorische Bewegung des Produkts 3 durchgeführt wird.

Bei all diesen Varianten ergeben sich durch die Ausbildung der Röntgenstrahlungserzeugungsvorrichtung 11 derart, dass die Bewegung der Röntgenstrahlungsquelle 19 parallel zur Richtung des unteren Randstrahl 35b des fächerartigen Röntgenstrahls 35 erfolgt, die zuvor in Bezug auf die Ausführungsform nach den Fig. 1 bis 4 beschriebenen Vorteile.
- 1, 1': Vorrichtung zur Röntgenuntersuchung von bewegten Produkten
- 3: Produkt
- 5: Gehäuse
- 7: Gerätefuß
- 9: Fördervorrichtung / Bandförder
- 11: Röntgenstrahlungserzeugungsvorrichtung
- 13: Linear-Verschiebevorrichtung
- 15: Detektorvorrichtung
- 17: Sensor
- 19: Röntgenstrahlungsquelle
- 21: Hochspannungsquelle
- 23: Trägerrahmen
- 25: Motor
- 25a: Antriebsriemen
- 27: Gewindespindel
- 29: Schlitten
- 31: Fördermittel / Endlosband
- 33: fächerartiger Röntgenstrahl
- 35a: oberer Randstrahl
- 35b: unterer Randstrahl
- 37: Bandkörper
- 39: Schenkelwandung
- 41: Dachwandung
- 42: Durchbruch
- 43: Ausnehmung
- 45: plattenförmiges Element
- 47: Nut
- 49: Auswerte-und Steuereinheit
- 53: Querwandungen
- 55: Bodenwandung
- 57: Strahlenschutzgehäuse
- 59: Deckel
- 61: Bewegungskurve
- h₀: minimale Höhe
- hₘₐₓ: maximale Höhe

- R: Förderrichtung
- E: Bewegungsebene

## Patentansprüche

1. Vorrichtung zur Röntgenuntersuchung von bewegten Produkten, insbesondere bewegten Stückgütern,
(a) mit einer Fördervorrichtung (9) mit einem Fördermittel (31), auf welchem ein zu durchstrahlendes Produkt (3) in einer Bewegungsebene (E) aufliegt und welches so ausgebildet ist, dass das Produkt entlang einer vorgegebenen Bewegungskurve mit einer vorgegebenen Geschwindigkeit oder entsprechend eines vorgebebenen zeitabhängigen Geschwindigkeits- oder Positionsverlaufs transportiert und/oder um eine im Wesentlichen senkrecht zur Bewegungsebene (E) stehende Rotationsachse rotiert wird,
(b) mit einer Strahlungserzeugungsvorrichtung (11) zur Erzeugung eines Röntgenstrahls (33), welche auf einer Seite des Fördermittels (31) vorgesehen ist, und mit einer Detektorvorrichtung (15), welche auf der gegenüberliegenden Seite des Fördermittels (31) vorgesehen ist,
(c) wobei die Strahlungserzeugungsvorrichtung (11) so angeordnet und ausgebildet ist, dass ein sich ausgehend von einer Röntgenstrahlungsquelle (19) der Strahlungserzeugungsvorrichtung (11) fächerartig erweiternder Röntgenstrahl (33) erzeugt wird, welcher einen oberen (35a) und einen unteren (35b) Randstrahl aufweist und welcher das Produkt (3) durchstrahlt, und
(d) wobei die Detektorvorrichtung (15) den auf sie auftreffenden Röntgenstrahl (33) erfasst und in ein Signal wandelt, welches einer Auswerte- und Steuereinheit (49) zugeführt ist,
**dadurch gekennzeichnet,**
(e) dass der untere Randstrahl (35b) des Röntgenstrahls (33) schräg zur Bewegungsebene (E)verläuft und
(f) dass die Röntgenstrahlungsquelle (19) in einer Richtung parallel zur Richtung des unteren Randstrahls (35b) des fächerartigen Röntgenstrahls linear verschiebbar ausgebildet ist.

2. Vorrichtung nach Anspruch 1, **dadurch gekennzeichnet, dass** die Röntgenstrahlungsquelle (19) so ausgebildet und angeordnet ist, dass der Röntgenstrahl (33) das Fördermittel (31) in der gesamten Breite des Fördermittels (31) durchdringt.

3. Vorrichtung nach Anspruch 1, **dadurch gekennzeichnet, dass** die Röntgenstrahlungsquelle (19) so ausgebildet und angeordnet ist, dass der Röntgenstrahl (33) das Fördermittel (31) in einer Teilbreite ausgehend von der der Detektorvorrichtung (15) zugewandten Seite bis zu einem nicht durchstrahlten Randbereich an der gegenüberliegenden Seite des Fördermittels (31) durchdringt.

4. Vorrichtung nach einem der vorhergehenden Ansprüche, **dadurch gekennzeichnet, dass** die Fördervorrichtung (9) ein Bandförderer und das Fördermittel (31) ein Obertrum eines angetriebenes Endlosbandes ist.

5. Vorrichtung nach Anspruch 4, **dadurch gekennzeichnet, dass** die Röntgenstrahlungsquelle (19) so ausgebildet und angeordnet ist, dass der untere Randstrahl (35a) des Röntgenstrahl (33) das Untertrum des Endlosbandes (31) nicht durchdringt.

6. Vorrichtung nach Anspruch 4 oder 5, **dadurch gekennzeichnet, dass** der Bandförderer (9) einen Bandkörper (37) aus einem Röntgenstrahlung reflektierenden und/oder stark absorbierenden Material umfasst, dass das Obertrum des Endlosbandes (31) auf einer Auflagefläche des Bandkörpers (37) aufliegt, und dass der Bandkörper (37) im Bereich des Röntgenstrahls (33) eine einschnittartige Ausnehmung (43) aufweist, durch welche der Röntgenstrahl (33) verläuft.

7. Vorrichtung nach Anspruch 6, **dadurch gekennzeichnet, dass** die Fördereinrichtung (9) zumindest im Bereich der Auflagefläche mit einem Material gefüllt ist, welches die Röntgenstrahlung im Wesentlichen vollständig oder nur mit geringer Dämpfung transmittiert, wobei vorzugsweise das Material im Bereich der Ausnehmung (43) einen Teil der Auflagefläche bildet.

8. Vorrichtung nach Anspruch 6 oder 7, **dadurch gekennzeichnet, dass** zumindest ein Teilbereich der Außenwandung des Bandkörpers (37) im Bereich der einschnittartigen Ausnehmung (43), welche nicht von der Ausnehmung (43) betroffen ist, einen Teil einerWandung eines Strahlenschutzgehäuses (57) wirkt.

9. Vorrichtung nach einem der Ansprüche 6 bis 8, **dadurch gekennzeichnet, dass** der Bandkörper (37) ein im Wesentlichen U-förmiges Profilstück mit zwei Schenkelwandungen (39) und einer die Auflagefläche bildenden, die Schenkelwandungen (39) verbindenden Dachwandung (41) umfasst.

10. Vorrichtung nach Anspruch 9, **dadurch gekennzeichnet, dass** das U-förmige Profilstück durch Biegen eines Röntgenstrahlung reflektierenden und/oder stark absorbierenden, vorzugsweise im Wesentlichen ebenen, plattenförmigen Teils (45), beispielsweise eines Metallblechteils, hergestellt ist, welches eine die Ausnehmung (43) für den Röntgenstrahl (33) bildenden Durchbruch aufweist.

11. Vorrichtung nach Anspruch 10, **dadurch gekennzeichnet, dass** das U-förmige Profilstück zwei Querwandungen (53) aus einem Röntgenstrahlung reflektierenden und/oder stark absorbierenden Material, vorzugsweise zwei Blechwandungen, aufweist, wobei eine erste Querwandung (53), in Förderrichtung (R) gesehen, vor der Ausnehmung (43) und eine zweite Querwandung (53) nach der Ausnehmung (43) für den Röntgenstrahl (33) vorgesehen ist, und wobei eine Bodenwandung (55) zusammen mit den Querwandungen (53) und jeweils vor und nach der Ausnehmung liegenden Bereichen der Dachwandung (41) eine Strahlungsfalle zum Absorbieren einer wesentlichen Menge der Röntgenstrahlung des fächerartigen Strahls (33) aufweist, welche die Ausnehmung (43) nicht auf einem im Wesentlichen geraden Weg durchdringt.

12. Vorrichtung nach Anspruch 3 und einem der Ansprüche 6 bis 11, **dadurch gekennzeichnet, dass** der Bandkörper (37) in seiner Auflagefläche eine in Förderrichtung (R) verlaufende Nut (47) aufweist, welche in einem seitlichen Randbereich der Auflagefläche verläuft, welcher, in Förderrichtung (R) gesehen, nicht mehr durch die Ausnehmung (43) unterbrochen ist, wobei in die Nut (47) ein Eingreifmittel des Endlosbandes, vorzugsweise eine in Förderrichtung (R) verlaufenden Erhebung an der Unterseite des Endlosbandes (31), eingreift, um Verschiebekräfte, die in einer Richtung quer zur Förderrichtung (R) auf das Obertrum des Endlosbandes (31) wirken, aufzunehmen.

13. Vorrichtung nach einem der vorhergehenden Ansprüche, **dadurch gekennzeichnet, dass** die Röntgenstrahlungsquelle (19) zusammen mit einer Hochspannungsquelle (21) der Strahlungserzeugungsvorrichtung (11) mittels einer Linear-Verschiebevorrichtung (13) linear verschiebbar ausgebildet ist.

14. Vorrichtung nach einem der vorhergehenden Ansprüche, **dadurch gekennzeichnet, dass** die Strahlungserzeugungsvorrichtung (11) zusammen mit der Linear-Verschiebevorrichtung (13) als Einheit ausgebildet ist und dass die Einheit als Ganzes aus der Vorrichtung (1, 1') zur Röntgenuntersuchung demontierbar ist.

15. Vorrichtung nach einem der vorhergehenden Ansprüche, **dadurch gekennzeichnet, dass** die Röntgenstrahlungsquelle (19) so ausgebildet ist,
(a) dass der obere Randstrahl (35a) des fächerartigen Röntgenstrahls (33) horizontal verläuft, und dass die Auswerte- und Steuereinheit (49) so ausgebildet ist, dass sie die Linear-Verschiebevorrichtung (13) so ansteuert, dass der obere Randstrahl (35a) im Wesentlichen mit einer vorgegebenen Höhenposition, insbesondere der Höhe, des jeweils zu untersuchenden Produkts (3) übereinstimmt, oder
(b) dass der fächerartige Röntgenstrahls (33) einen horizontal verlaufenden Teilstrahl aufweist, und dass die Auswerte- und Steuereinheit (49) so ausgebildet ist, dass sie die Linear-Verschiebevorrichtung (13) so ansteuert, dass der Teilstrahl im Wesentlichen mit einer vorgegebenen Höhenposition, insbesondere der Höhe, des jeweils zu untersuchenden Produkts (3) übereinstimmt.

## Claims

1. A device for X-ray examination of moving products, in particular moving piece goods,
(a) having a conveying device (9) with a conveying means (31) on which a product (3) that is to be irradiated rests in a movement plane (E) and which is formed in such a way that the product is transported along a predetermined curve of movement at a predetermined speed or correspondingly a predetermined time-dependent course of speed or position and/or is rotated about a rotational axis that is substantially perpendicular to the movement plane (E),
(b) having a radiation-generating device (11) for generating an X-ray beam (33) provided on one side of the conveying means (31), and having a detector device (15) provided on the opposite side of the conveying means (31),
(c) wherein the radiation-generating device (11) is arranged and formed in such a way that an X-ray beam (33) is generated that widens in a fan-like manner starting from an X-ray radiation source (19) of the radiation-generating device (11), has an upper marginal beam (35a) and a lower marginal beam (35b) and irradiates the product (3), and
(d) wherein the detector device (15) detects the X-ray beam (33) impinging on it and converts it into a signal which is fed to an evaluation and control unit (49),
**characterised in that**
(e) the lower marginal beam (35b) of the X-ray beam (33) extends obliquely with respect to the movement plane (E), and
(f) the X-ray radiation source (19) is formed so that it is linearly displaceable in a direction parallel to the direction of the lower marginal beam (35b) of the fan-like X-ray beam.

2. A device according to claim 1, **characterised in that** the X-ray radiation source (19) is formed and arranged in such a way that the X-ray beam (33) penetrates the conveying means (31) throughout the width of the conveying means (31).

3. A device according to claim 1, **characterised in that** the X-ray radiation source (19) is formed and arranged in such a way that the X-ray beam (33) penetrates the conveying means (31) in a partial width starting from the side facing the detector device (15) as far as a marginal region that is not irradiated on the opposite side of the conveying means (31).

4. A device according to one of the preceding claims, **characterised in that** the conveying device (9) is a belt conveyor, and the conveying means (31) is an upper run of a driven endless belt.

5. A device according to claim 4, **characterised in that** the X-ray radiation source (19) is formed and arranged in such a way that the lower marginal beam (35a) of the X-ray beam (33) does not penetrate the lower run of the endless belt (31).

6. A device according to claim 4 or 5, **characterised in that** the belt conveyor (9) comprises a belt body (37) made from a material that reflects and/or strongly absorbs X-ray radiation, the upper run of the endless belt (31) rests on a support surface of the belt body (37), and the belt body (37) has in the region of the X-ray beam (33) a notch-like recess (43) through which the X-ray beam (33) extends.

7. A device according to claim 6, **characterised in that** the conveying device (9) is filled at least in the region of the support surface with a material which transmits the X-ray radiation substantially completely or only with little damping, with the material in the region of the recess (43) preferably forming a portion of the support surface.

8. A device according to claim 6 or 7, **characterised in that** at least a partial region of the outer wall of the belt body (37) in the region of the notch-like recess (43), which is unaffected by the recess (43), effects a portion of a wall of a radiation-protection housing (57).

9. A device according to one of claims 6 to 8, **characterised in that** the belt body (37) comprises a substantially U-shaped profiled piece with two side walls (39) and a top wall (41) forming the support surface and connecting the side walls (39).

10. A device according to claim 9, **characterised in that** the U-shaped profiled piece is produced by bending a preferably substantially planar, plate-shaped portion (45), for example a metal-sheet portion, that reflects and/or strongly absorbs X-ray radiation and has an opening forming the recess (43) for the X-ray beam (33).

11. A device according to claim 10, **characterised in that** the U-shaped profiled piece has two transverse walls (53) made from a material that reflects and/or strongly absorbs X-ray radiation, preferably two sheet-metal walls, wherein a first transverse wall (53), viewed in the conveying direction (R), is provided upstream of the recess (43), and a second transverse wall (53) is provided downstream of the recess (43) for the X-ray beam (33), and wherein a base wall (55) together with the transverse walls (53) and regions of the top wall (41), lying respectively upstream and downstream of the recess, has a radiation trap to absorb a substantial quantity of the X-ray radiation of the fan-like beam (33) which does not penetrate the recess (43) along a substantially straight path.

12. A device according to claim 3 and one of claims 6 to 11, **characterised in that** the belt body (37) has in its support surface a groove (47) which extends in the conveying direction (R) and extends in a lateral marginal region of the support surface which, viewed in the conveying direction (R), is no longer interrupted by the recess (43), wherein an engagement means of the endless belt, preferably an elevation that extends in the conveying direction (R) on the underside of the endless belt (31), engages into the groove (47) in order to take up displacement forces which act on the upper run of the endless belt (31) in a direction transversely to the conveying direction (R).

13. A device according to one of the preceding claims, **characterised in that** the X-ray radiation source (19), together with a high-voltage source (21) of the radiation-generating device (11), is formed so that it is linearly displaceable by means of a linear-displacement device (13)

14. A device according to one of the preceding claims, **characterised in that** the radiation-generating device (11) is formed as a unit together with the linear-displacement device (13), and **in that** the unit can be disassembled as a whole from the device (1, 1') for X-ray examination.

15. A device according to one of the preceding claims, **characterised in that** the X-ray radiation source (19) is formed in such a way
(a) that the upper marginal beam (35a) of the fan-like X-ray beam (33) extends horizontally, and that the evaluation and control device (49) is formed in such a way that it activates the linear-displacement device (13) in such a way that the upper marginal beam (35a) corresponds substantially with a predetermined height position, in particular the height, of the product (3) that is to be examined in each case, or
(b) that the fan-like X-ray beam (33) has a horizontally extending partial beam, and that the evaluation and control unit (49) is formed in such a way that it activates the linear-displacement device (13) in such a way that the partial beam corresponds substantially with a predetermined height position, in particular the height, of the product (3) that is to be examined in each case.

## Revendications

1. Dispositif d'examen radiologique de produits mis en mouvement, en particulier de marchandises de détail mises en mouvement,
(a) avec un dispositif de transport (9) avec un moyen de transport (31) sur lequel un produit (3) à irradier repose dans un plan de mouvement (E) et qui est conçu de telle sorte que le produit est transporté le long d'une courbe de déplacement prédéterminée avec une vitesse prédéterminée ou correspondant à une évolution de la vitesse ou de la position en fonction du temps prédéterminée, et/ou tourne autour d'un axe de rotation sensiblement perpendiculairement au plan de mouvement (E),
(b) avec un dispositif de génération de rayonnement (11) pour générer un faisceau de rayons X (33) qui est prévu sur un côté du moyen de transport (31), et avec un dispositif détecteur (15) qui est prévu sur le côté opposé du moyen de transport (31),
(c) dans lequel le dispositif de génération de rayonnement (11) est agencé et conçu de telle sorte qu'un faisceau de rayons X (33) s'élargissant en éventail à partir d'une source de rayonnement de rayons X (19) du dispositif de génération de rayonnement (11) est généré, qui présente un rayonnement de bordure supérieur (35a) et inférieur (35b) et qui irradie le produit (3), et
(d) dans lequel le dispositif détecteur (15) détecte le faisceau de rayons X (33) incident et le transforme en un signal qui est transmis à une unité d'évaluation et de commande (49),
**caractérisé en ce que**
(e) le rayonnement de bordure inférieur (35b) du faisceau de rayons X (33) s'étend en biais au plan de mouvement (E) et
(f) la source de rayonnement de rayons X (19) est conçue de façon à pouvoir être décalée linéairement dans une direction parallèle à la direction du rayonnement de bordure inférieur (35b) du faisceau de rayons X en éventail.

2. Dispositif selon la revendication 1, **caractérisé en ce que** la source de rayonnement de rayons X (19) est conçue et agencée de telle sorte que le faisceau de rayons X (33) traverse le moyen de transport (31) dans toute la largeur du moyen de transport (31).

3. Dispositif selon la revendication 1, **caractérisé en ce que** la source de rayonnement de rayons X (19) est conçue et agencée de telle sorte le faisceau de rayons X (33) traverse le moyen de transport (31) dans une largeur partielle à partir du côté tourné vers le dispositif détecteur (15) jusqu'à une zone périphérique non irradiée sur le côté opposé du moyen de transport (31).

4. Dispositif selon l'une des revendications précédentes, **caractérisé en ce que** le dispositif de transport (9) est un transporteur à bande et le moyen de transport (31) est un brin supérieur d'une courroie sans fin entraînée.

5. Dispositif selon la revendication 4, **caractérisé en ce que** la source de rayonnement de rayons X (19) est conçue et agencée de telle sorte que le rayonnement de bordure inférieur (35a) du faisceau de rayons X (33) ne traverse pas le brin inférieur de la courroie sans fin (31).

6. Dispositif selon la revendication 4 ou 5, **caractérisé en ce que** le transporteur à bande (9) comprend un corps de bande (37) en un matériau réfléchissant et/ou absorbant fortement un rayonnement de rayons X, **en ce que** le brin supérieur de la courroie sans fin (31) repose sur une surface d'appui du corps de bande (37) et **en ce que** le corps de bande (37) présente, dans la zone du faisceau de rayons X (33), un évidement (43) de type incision par lequel s'étend le faisceau de rayons X (33).

7. Dispositif selon la revendication 6, **caractérisé en ce que** le dispositif de transport (9) est rempli, au moins dans la zone de la surface d'appui, avec un matériau qui transmet le rayonnement de rayons X sensiblement complètement ou seulement avec une faible atténuation, dans lequel de préférence le matériau forme, dans la zone de l'évidement (43), une partie de la surface d'appui.

8. Dispositif selon la revendication 6 ou 7, **caractérisé en ce qu'**au moins une zone partielle de la paroi externe du corps de bande (37) agit dans la zone de l'évidement (43) de type incision qui n'est pas concernée par l'évidement (43), sur une partie d'une paroi d'un boîtier de protection contre le rayonnement (57).

9. Dispositif selon l'une des revendications 6 à 8, **caractérisé en ce que** le corps de bande (37) comprend un profilé sensiblement en forme de U avec deux parois latérales (39) et une paroi de toit (41) formant la surface d'appui, reliant les parois latérales (39).

10. Dispositif selon la revendication 9, **caractérisé en ce que** le profilé en forme de U est fabriqué par courbure d'une partie réfléchissant et/ou absorbant fortement un rayonnement de rayons X, de préférence sensiblement plane, sous forme de plaque (45), par exemple une partie de tôle métallique, qui présente une perforation formant l'évidement (43) pour le faisceau de rayons X (33).

11. Dispositif selon la revendication 10, **caractérisé en ce que** le profilé en forme de U présente deux parois transversales (53) en un matériau réfléchissant et/ou absorbant fortement un rayonnement de rayons X, de préférence deux parois en tôle, dans lequel une première paroi transversale (53), vue dans la direction de transport (R), est prévue avant l'évidement (43) et une deuxième paroi transversale (53) est prévue après l'évidement (43) pour le faisceau de rayons X (33), et dans lequel une paroi de fond (55) conjointement avec les parois transversales (53) et des zones, se trouvant respectivement avant et après l'évidement, de la paroi de toit (41) présente un piège de rayonnement pour l'absorption d'une quantité sensible du rayonnement de rayons X du faisceau (33) en éventail qui ne traverse pas l'évidement (43) d'une façon qui est sensiblement linéaire.

12. Dispositif selon la revendication 3 et l'une des revendications 6 à 11, **caractérisé en ce que** le corps de bande (37) présente, dans sa surface d'appui, une rainure (47) s'étendant dans la direction de transport (R) qui s'étend dans une zone périphérique latérale de la surface d'appui, qui, vue dans la direction de transport (R), n'est plus interrompue par l'évidement (43), dans lequel un moyen de mise en prise de la courroie sans fin, de préférence une protubérance s'étendant dans la direction de transport (R) sur la face inférieure de la courroie sans fin (31), vient en prise dans la rainure (47) pour absorber des forces de décalage qui agissent dans une direction transversalement à la direction de transport (R) sur le brin supérieur de la courroie sans fin (31).

13. Dispositif selon l'une des revendications précédentes, **caractérisé en ce que** la source de rayonnement de rayons X (19) est conçue de façon à pouvoir être décalée linéairement, conjointement avec une source de haute tension (21) du dispositif de génération de rayonnement (11) au moyen d'un dispositif de décalage linéaire (13).

14. Dispositif selon l'une des revendications précédentes, **caractérisé en ce que** le dispositif de génération de rayonnement (11) est conçu, conjointement avec le dispositif de décalage linéaire (13), comme une unité et **en ce que** l'unité dans son ensemble peut être démontée du dispositif (1, 1') pour l'examen radiologique.

15. Dispositif selon l'une des revendications précédentes, **caractérisé en ce que** la source de rayonnement de rayons X (19) est conçue de telle sorte
(a) que le rayonnement de bordure supérieur (35a) du faisceau de rayons X (33) en éventail s'étend horizontalement et que l'unité d'évaluation et de commande (49) est conçue de telle sorte qu'elle commande le dispositif de décalage linéaire (13) de sorte que le rayonnement de bordure supérieur (35a) corresponde sensiblement à une position en hauteur prédéterminée, en particulier à la hauteur du produit (3) respectif à examiner, ou
(b) que le faisceau de rayons X (33) en éventail présente un faisceau partiel s'étendant horizontalement et que l'unité d'évaluation et de commande (49) est conçue de telle sorte qu'elle commande le dispositif de décalage linéaire (13) de sorte que le faisceau partiel corresponde sensiblement à une position en hauteur prédéterminée, en particulier à la hauteur du produit (3) respectif à examiner.
